(19)

Europäisches
Patentamt
European
Patent Office
Office européen
des brevets

(11)  **EP 3 164 069 B1**

(12)  **EUROPEAN PATENT SPECIFICATION**

(45) Date of publication and mention
of the grant of the patent:
**16.09.2020  Bulletin 2020/38**

(21) Application number: **15814955.9**

(22) Date of filing: **03.07.2015**

(51) Int Cl.:
***A61B 5/055*** (2006.01)

(86) International application number:
**PCT/US2015/039162**

(87) International publication number:
**WO 2016/004423 (07.01.2016 Gazette 2016/01)**

(54) **SYSTEMS AND METHODS FOR IDENTIFYING AND PROFILING MUSCLE PATTERNS**

SYSTEME UND VERFAHREN ZUR IDENTIFIZIERUNG UND PROFILIERUNG VON
MUSKELSTRUKTUREN

SYSTÈMES ET PROCÉDÉS D'IDENTIFICATION ET DE PROFILAGE DE PROFILS MUSCULAIRES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority:  **03.07.2014  US 201462020779 P**

(43) Date of publication of application:
**10.05.2017  Bulletin 2017/19**

(73) Proprietor: **University Of Virginia Patent
Foundation
Charlottesville, VA 22903 (US)**

(72) Inventors:
 • **HANDSFIELD, Geoffrey G.
Charlottesvile, VA 22903 (US)**
 • **KNAUS, Katherine R.
Charlottesville, VA 22903 (US)**
 • **BLEMKER, Silvia S.
Charlottesville, VA 22903 (US)**
 • **MEYER, Craig H.
Charlottesville, VA 22903 (US)**
 • **HART, Joseph M.
Charlottesville, VA 22903 (US)**

(74) Representative: **HGF
1 City Walk
Leeds LS11 9DX (GB)**

(56) References cited:
**WO-A1-2013/023214    US-A1- 2008 195 322
US-A1- 2014 364 723    US-B2- 7 949 180**

• **HANDSFIELD GEOFFREY G ET AL:
"Relationships of 35 lower limb muscles to height
and body mass quantified using MRI", JOURNAL
OF BIOMECHANICS, PERGAMON PRESS, NEW
YORK, NY, US, vol. 47, no. 3, 11 December 2013
(2013-12-11), pages 631-638, XP028812603, ISSN:
0021-9290, DOI:
10.1016/J.JBIOMECH.2013.12.002**
• **NISHIZAWA H ET AL: "HIERARCHICAL
CLUSTERING METHOD FOR THE ANALYSIS OF
LARGE AMOUNT OF DATA", VISUAL
COMMUNICATIONS AND IMAGE PROCESSING;
20-1-2004 - 20-1-2004; SAN JOSE,, vol. 2824, 1
January 1996 (1996-01-01), pages 183-190,
XP000905399, DOI: 10.1117/12.258130 ISBN:
978-1-62841-730-2**
• **Anonymous: "2013 Medical Imaging Technical
Summaries - excerpts from 8670:
Computer-Aided Diagnosis", 2013 Medical
Imaging, 9 February 2013 (2013-02-09), pages
93-125, XP055412935, Retrieved from the
Internet:
URL:http://spie.org/conferences-and-exhibi
tions/past-conferences-and-exhibitions/med
ical-imaging-2013?SSO=1 [retrieved on
2017-10-05]**

EP 3 164 069 B1

**EP 3 164 069 B1**

**Description**

Some references, which may include patents, patent applications, and various publications, are cited in a reference list and discussed in the disclosure provided herein. The citation and/or discussion of such references is provided merely to clarify the description of the present disclosure and is not an admission that any such reference is "prior art" to any aspects of the present disclosure described herein. In terms of notation, hereinafter, "[n]" represents the $n^{th}$ reference cited in the reference list. For example, [3] represents the third reference cited in the reference list, namely Meyer, C. H., et al., Simultaneous spatial and spectral selective excitation. Magnetic Resonance in Medicine 15: 287-304 (1990).

BACKGROUND

Movements of the human body have long been the source of scientific fascination, which has led to widespread curiosity about the skeletal muscles responsible for athletic movement, particularly in the lower limb. In order to excel, athletes must train extensively. Muscle has an incredible aptitude for adaptation, and the question of what adaptation looks like when induced by athletic training has motivated numerous studies focused on quantifying anatomical changes in skeletal muscle due to athletic training. Past approaches to answering this question have involved comparing athletes to untrained people and other athletes in different sports, longitudinally measuring subjects before and after training regimens, and even comparing between different types of training. Most of these studies measured differences in muscle size, or the amount of hypertrophy.

With the use of imaging modalities, muscle size can be measured *in vivo,* commonly by computing the cross sectional area (CSA) of a muscle, or several, in one or more locations. This method has been favored because data can be collected relatively quickly, by only identifying anatomy in a couple of images. Muscle volumes can be measured similarly by summing CSA from continuous transverse images and multiplying by slice thickness. Volume is a more appropriate *in vivo* metric of muscle function than the CSA in a single transverse image, because the volume determines the power generating capacity of a muscle, and can be used to calculate its physiological cross sectional area (PCSA), which determines the muscle force.

The time demands of the imaging and computation required for measuring muscle volumes have limited many studies to focus on only a few subjects and a few muscles, primarily in the quadriceps or triceps surae. The hypertrophy measured in athletes or after athletic training is not uniform across groups of several muscles or even in different regions of the same muscle. Athletes do not commonly train single muscles, or even functional groups, in isolation, but rather involve the whole limb. While it has been assumed that adaptations induced by this training are not evenly distributed across the muscles of the lower limb, and instances of non-uniformity have been revealed in previous research, a comprehensive assessment of patterns of muscle adaptation across the entire lower limb has not yet been conducted for athletes. It is known that athletes exhibit greater performance than healthy non-athletes, which is linked in part to muscle hypertrophy, but the patterns of hypertrophy across muscles and how those patterns relate to athlete performance has been relatively unknown.

It is with respect to these and other considerations that the various embodiments described below are presented.

WO2013/023214 A1 describes a method for identifying muscle abnormality. The method includes acquiring image data associated with a plurality of muscles and generating a data model for the plurality of muscles based on the image data. The method further includes calculating the volume and/or length of the plurality of muscles based on the data model, and determining if the volume and/or length for the muscle, as calculated, deviates from volume and/or length associated with a healthy muscle. If it is determined that the volume and/or length for the muscle deviates from the volume and/or length associated with a healthy muscle, a muscle abnormality can be identified based on the deviation.

SUMMARY

The present invention relates to a method, a system and a computer-readable medium for identifying and profiling muscle patterns, as defined in the independent claims.

In one aspect, the present disclosure relates to a method that, in one embodiment, includes acquiring image data associated with a selected muscle or group of muscles of one or more subjects and determining, based on the image data, muscle volume of the selected muscle or group of muscles. The method also includes calculating, based on the muscle volume and the height and mass of the one or more subjects, a height-mass normalized muscle volume for the selected muscle or group of muscles, and determining a deviation of the height-mass normalized muscle volume of the selected muscle or group of muscles from a mean value of muscle volume associated with a corresponding reference muscle or reference group of muscles. The method also includes identifying, based on the deviation, a muscle abnormality or absence of a muscle abnormality in the selected muscle or group of muscles.

In another aspect, the present disclosure relates to a system that, in one embodiment, includes a data acquisition device and a processing device. The data acquisition device is configured to acquire image data associated with a selected muscle or group of muscles of one or more subjects. The processing device is configured to perform functions that include determining, based on the image data, muscle volume of the selected muscle or group of muscles, and calculating, based on the muscle volume and the height and mass of the one or more subjects, a height-mass normalized muscle volume for the selected muscle or group of muscles. The processing device is also configured to determine a deviation of the height-mass normalized muscle volume of the selected muscle or group of muscles from a mean value of muscle volume associated with a corresponding reference muscle or reference group of muscles. The processing device is also configured to identify, based on the deviation, a muscle abnormality or absence of a muscle abnormality in the selected muscle or group of muscles.

In yet another aspect, the present disclosure relates to a non-transitory computer-readable medium which, in one embodiment, has stored computer-executable instructions that, when executed by one or more processors, cause a computing device to perform a method that includes acquiring image data associated with a selected muscle or group of muscles of one or more subjects and determining, based on the image data, muscle volume of the selected muscle or group of muscles. The method also includes calculating, based on the muscle volume and the height and mass of the one or more subjects, a height-mass normalized muscle volume for the selected muscle or group of muscles, and determining a deviation of the height-mass normalized muscle volume of the selected muscle or group of muscles from a mean value of muscle volume associated with a corresponding reference muscle or reference group of muscles. The method also includes identifying, based on the deviation, a muscle abnormality or absence of a muscle abnormality in the selected muscle or group of muscles.

Other aspects and features according to the present disclosure will become apparent to those of ordinary skill in the art, upon reviewing the following detailed description in conjunction with the accompanying figures.

BRIEF DESCRIPTION OF THE DRAWINGS

Reference will now be made to the accompanying drawings, which are not necessarily drawn to scale.

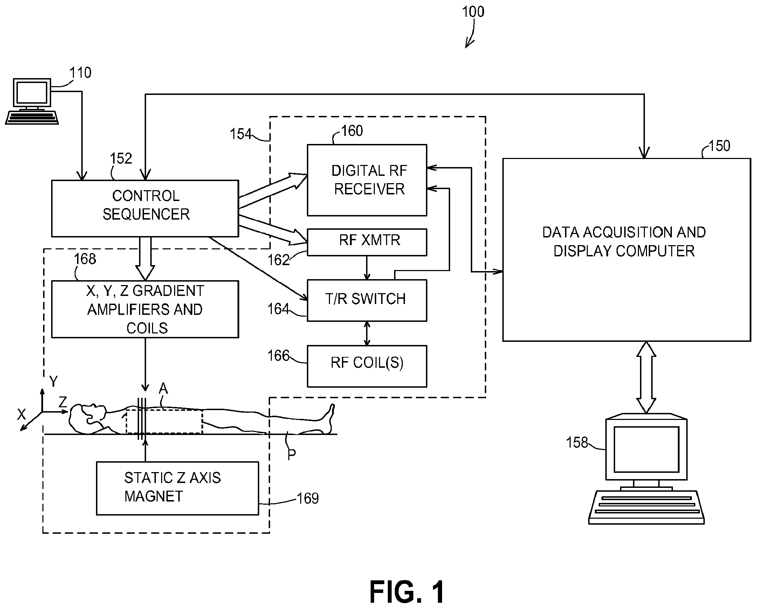

FIG. 1 is a system diagram illustrating an imaging system capable of implementing aspects of the present disclosure in accordance with one or more embodiments.

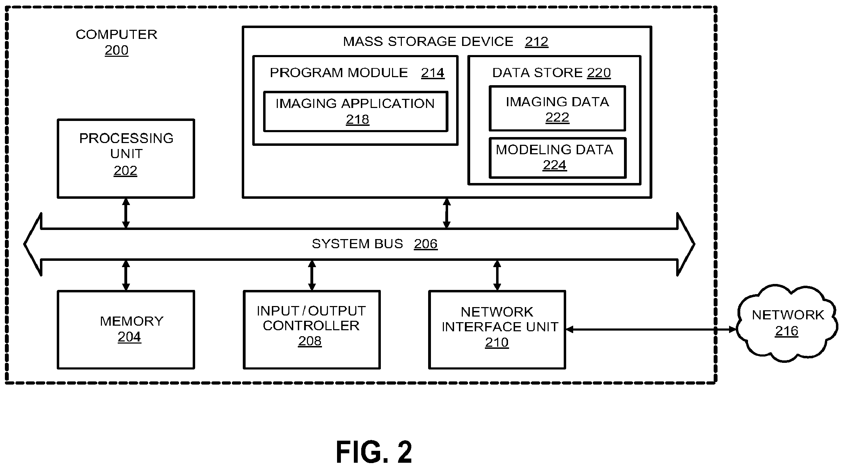

FIG. 2 is a computer architecture diagram showing a general computing system capable of implementing aspects of the present disclosure in accordance with one or more embodiments.

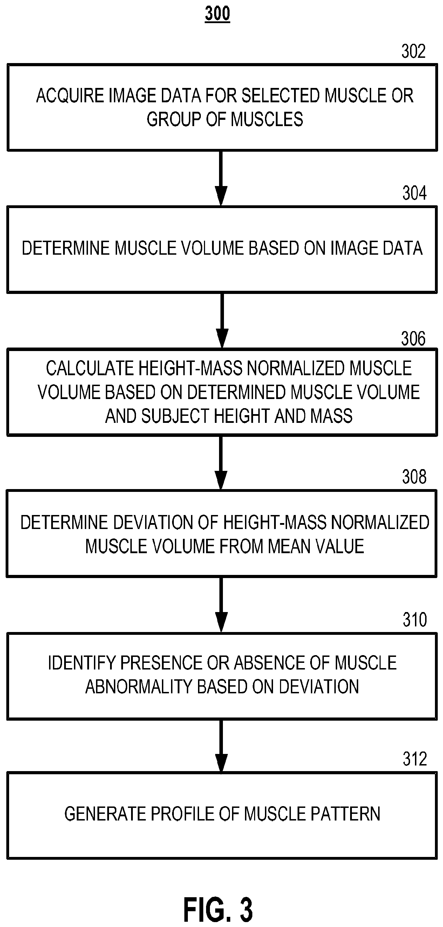

FIG. 3 is a flow diagram illustrating operations of a method for identifying and profiling muscle patterns according to one embodiment of the present disclosure.

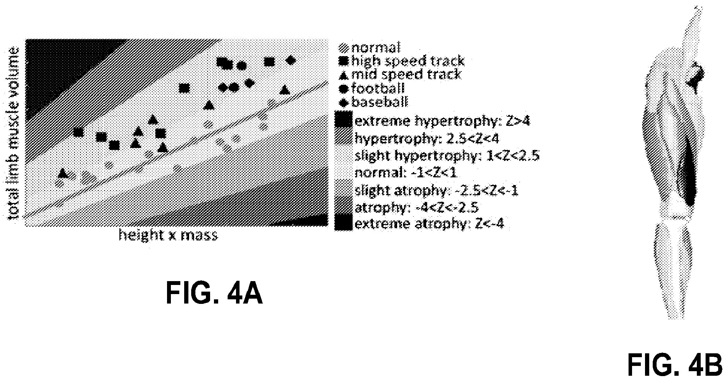

FIGS. 4A and 4B illustrate subject total limb muscle volumes plotted in color-coded regions of hypertrophy (FIG. 4A) and individual limb muscles that are color-coded by the amount of hypertrophy (FIG. 4B), in accordance with an example implementation of some aspects of the present disclosure that is also referred to herein as "Example 1".

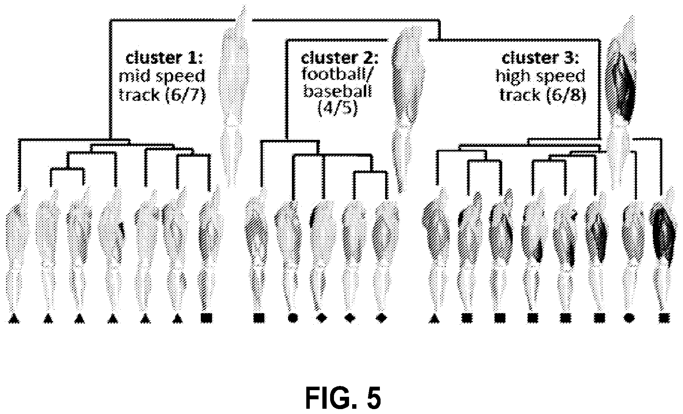

FIG. 5 illustrates, with respect to Example 1, a dendogram representation of individual athlete limbs that have been sorted based on 35-D clustering along muscle dimensions. The weighted mean limbs of three primary clusters of similar athletes are shown.

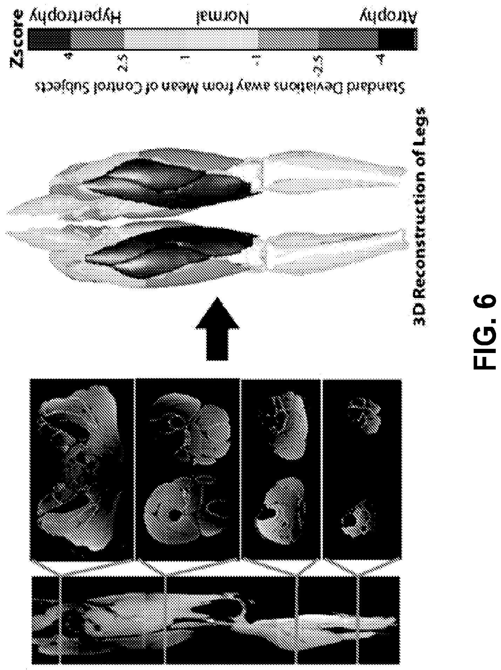

FIG. 6 illustrates 35 lower limb muscle volumes measured in magnetic resonance imaging (MRI) of athletes and compared to control subjects to quantify hypertrophy, in accordance with an example implementation of some aspects of the present disclosure that is also referred to herein as "Example 2". For each athlete, the volumes of 35 lower limb muscles were measured from MRI cross-sections, and these volumes were normalized by each athlete's body size (height*mass). Hypertrophy was calculated using Z scores to compare individual muscles to a database of healthy control subjects. As shown, each muscle is colored according to its hypertrophy Z score.

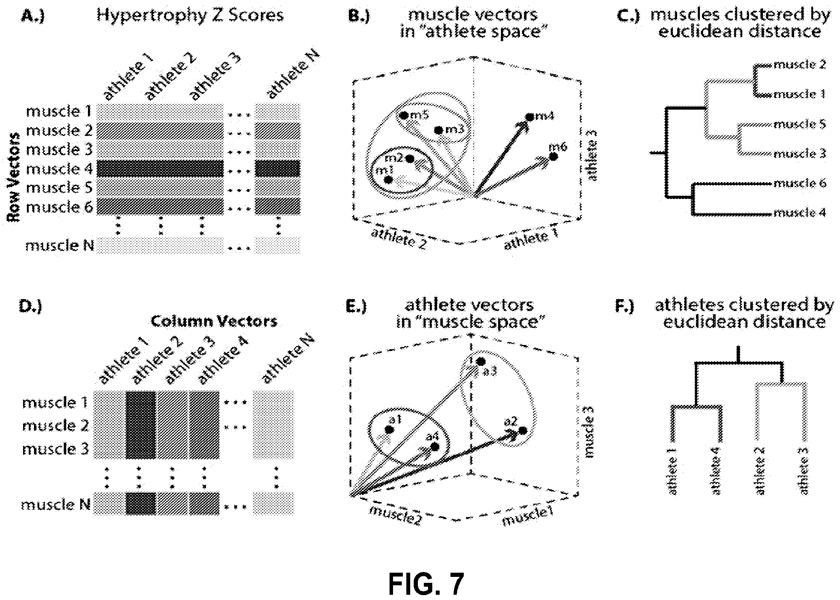

FIG. 7 illustrates, with respect to Example 2, various aspects of multi-dimensional hierarchical clustering used to reveal statistical similarities in hypertrophy between different athletes and their muscles. The Z scores representing hypertrophy are organized into a matrix with columns of athletes and rows of muscles. Muscle rows in "A." are plotted as vectors in "athlete space" in "B.", where only the first three dimensions (columns) are shown. Muscle vectors are clustered with the vector or group of vectors that is the shortest Euclidean distance away, forming a hierarchy in "C." Likewise, athlete columns shown in "D." are plotted as vectors in "muscle space" in "E." These athlete vectors are grouped by their Euclidean distance from each other, which compares vector magnitudes, shown in "F."

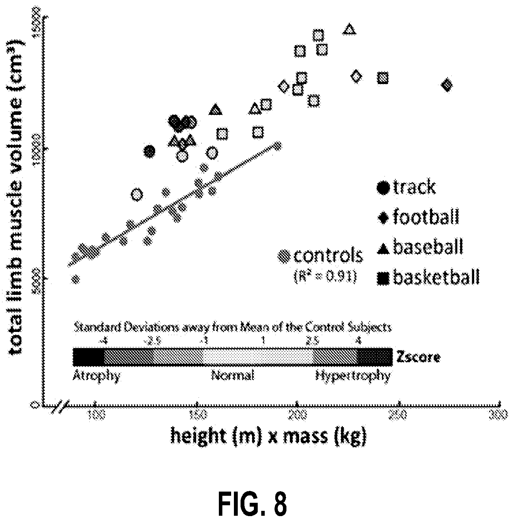

FIG. 8 illustrates, with respect to Example 2, the total volume of the 35 muscles in the dominant lower limb of each subject plotted against the product of their height and mass. Athlete points are colored by the Z score of their total volume per height*mass when compared to the control population. Z score indicates athletes' deviation from the scaling relationship between muscle volume and body size displayed by healthy non-athlete control subjects.

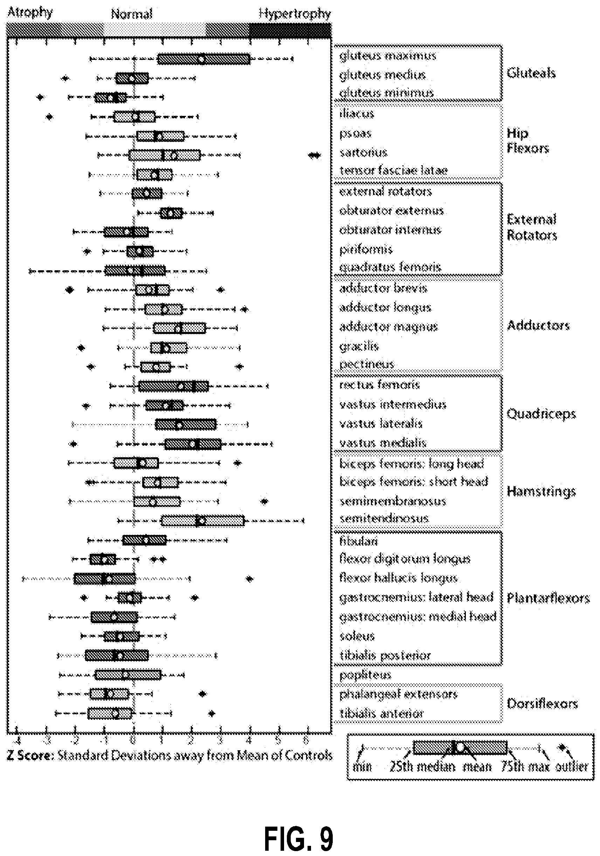

FIG. 9 illustrates, with respect to Example 2, hypertrophy across the group of 35 athletes for each lower limb muscle, where the circle represents the mean colored according to Z score, the central mark represents the median, the edges of the box represent the 25th and 75th percentile, and the whiskers extend to the most extreme data points, except for outliers which are plotted individually. The line at 0 represents the mean of the control subjects; muscles more than 1 standard deviation above are considered hypertrophic, and muscles more than 1 standard deviation below are considered atrophic.

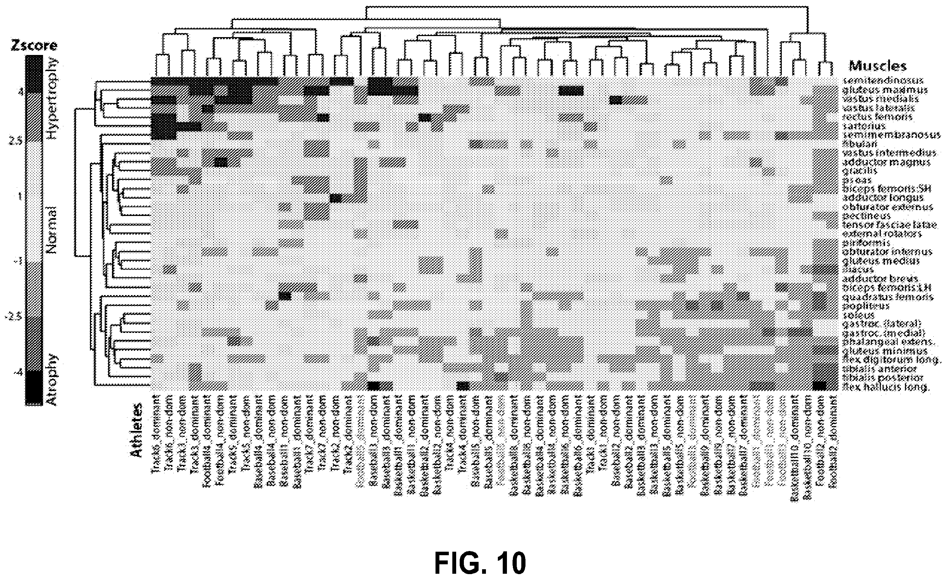

FIG. 10 illustrates, with respect to Example 2, hypertrophy measured for each muscle, colored accordingly and organized into columns corresponding to the individual legs of each athlete, with a row for each lower limb muscle. The muscles (rows) and athletes (columns) are clustered by Euclidean distance. The dendrograms (tree diagrams) show the hierarchical links between individual athletes and muscles. As will be discussed in further detail below, it can be seen from FIG. 10 that for most athletes, their dominant leg and non-dominant legs cluster together before grouping with another athlete's leg, meaning the hypertrophy of the two legs of an athlete are more statistically similar to each other than to other athletes. Shown in red, athletes whose legs do not cluster together have severe asymmetric conditions.

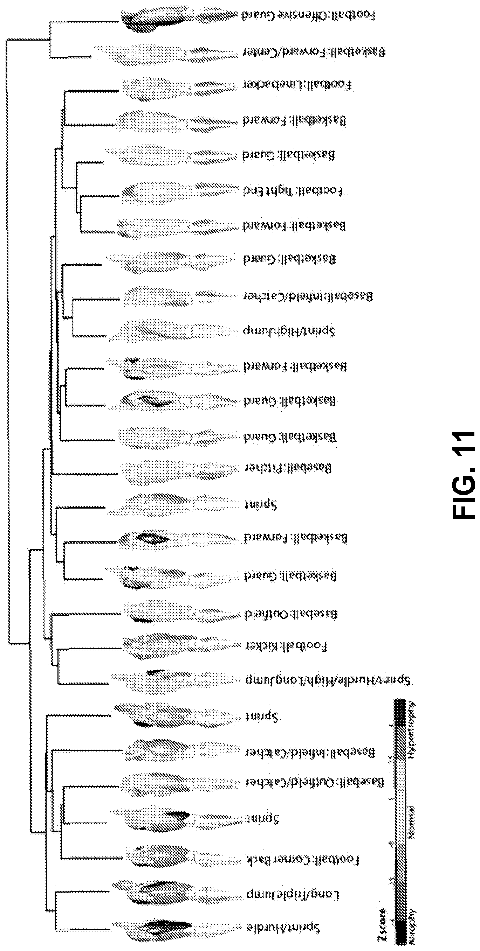

FIG.11 illustrates, with respect to Example 2, athletes clustered hierarchically with other athletes that have similar phenotypes, or patterns of hypertrophy. Athletes are clustered with only their dominant leg shown, with individual muscles colored according to hypertrophy Z score, illustrating each athlete's phenotype. Athletes are labeled by their respective sporting event or position.

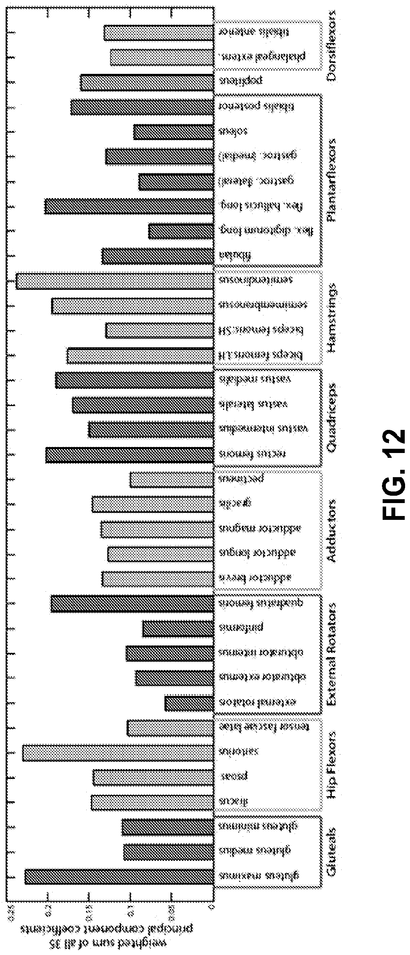

FIG. 12 illustrates, with respect to Example 2, representations of the weighted sums of component coefficients for particular muscles. The sum of all of the coefficients for each principal component are weighted according to the amount of overall variance described by that component. Each muscle's weighted coefficients from each of the principal components are summed. The sum of the muscle's weighted component coefficients reveals the sensitivity of the clustering to hypertrophy in that particular muscle. As will be discussed in further detail below, it can be seen from FIG. 12 that hypertrophy of muscles with the highest sum of their component coefficients most affect athlete clustering.

## DETAILED DESCRIPTION

Some aspects of the present disclosure relate to methods, systems, and computer-readable media for performing aspects of identifying and profiling muscle patterns. Although example embodiments of the present disclosure are explained in detail herein, it is to be understood that other embodiments are contemplated. Accordingly, it is not intended that the present disclosure be limited in its scope to the details of construction and arrangement of components set forth in the following description or illustrated in the drawings. The present disclosure is capable of other embodiments and of being practiced or carried out in various ways.

It must also be noted that, as used in the specification and the appended claims, the singular forms "a," "an" and "the" include plural referents unless the context clearly dictates otherwise. Ranges may be expressed herein as from "about" or "approximately" one particular value and/or to "about" or "approximately" another particular value. When such a range is expressed, other exemplary embodiments include from the one particular value and/or to the other particular value.

By "comprising" or "containing" or "including" is meant that at least the named compound, element, particle, or method step is present in the composition or article or method, but does not exclude the presence of other compounds, materials, particles, method steps, even if the other such compounds, material, particles, method steps have the same function as what is named.

In describing example embodiments, terminology will be resorted to for the sake of clarity. It is intended that each term contemplates its broadest meaning as understood by those skilled in the art and includes all technical equivalents that operate in a similar manner to accomplish a similar purpose. It is also to be understood that the mention of one or more steps of a method does not preclude the presence of additional method steps or intervening method steps between those steps expressly identified. Steps of a method may be performed in a different order than those described herein without departing from the scope of the present disclosure. Similarly, it is also to be understood that the mention of one or more components in a device or system does not preclude the presence of additional components or intervening components between those components expressly identified.

As discussed herein, a "subject" may be any applicable human subject, for example an athlete or normal healthy subject. Alternatively, a subject may be any animal. It should be appreciated that an animal may be a variety of any applicable type, including, but not limited thereto, mammal, veterinarian animal, livestock animal or pet type animal.

An overview of some objectives and example embodiments and implementations of the present disclosure will now be provided. In accordance with some embodiments, rapid non-Cartesian MRI and image processing is used to compile a comprehensive dataset of volumes of lower limb muscles in healthy non-athlete controls and in collegiate athletes actively competing in various varsity sports. As will be described in further detail below, an individual can have unique patterns of hypertrophy in their lower limb that can be used to establish a "phenotype", in order to compare individuals within a population of athletes. The hypertrophy of individual muscles can be quantified by comparing individual athletes to healthy control subjects to determine the distribution of hypertrophy across the lower limb of an athlete and to compare this distribution between athletes.

Clustering analysis is a data modeling tool which can compile large datasets in multi-dimensional space in order to group similar observations and variables. Clustering has been used to study phenotypes, which are unique patterns of gene expression in different conditions. In each of multiple experimental conditions, expression levels are measured for a large number of different genes, and these expression levels are compared across genes and across conditions in order to identify specific phenotypes, as well as to group the experimental conditions with the most similar pattern of gene expression and group the genes that express similarly in different conditions. The experimental conditions are the observations, the individual genes are the variables, and the measured parameter is the level of expression.

A comprehensive analysis of the distribution of hypertrophy in athletes' lower limbs can involve comparing the different muscles in a single athlete and then comparing across athletes. In accordance with some aspects of the present disclosure, clustering analysis may be applied to athletes in order to understand muscle hypertrophy, where the individual athletes are the observations, individual muscles are the variables, and the amount of hypertrophy is the measured parameter. Athlete phenotypes can be identified to indicate how athletes can differ within a particular sport and across an athlete population. By identifying muscles that clustering may be most sensitive to, the extent to which hypertrophy of particular muscles affects these phenotypes may be determined.

A further detailed description of aspects of the present disclosure will now be provided with reference to the accompanying drawings. The drawings form a part hereof and show, by way of illustration, specific embodiments or examples. In referring to the drawings, like numerals represent like elements throughout the several figures.

FIG. 1 is a system diagram illustrating an operating environment capable of implementing aspects of the present disclosure in accordance with one or more example embodiments. FIG. 1 illustrates an example of a magnetic resonance imaging (MRI) system 100, including a data acquisition and display computer 150 coupled to an operator console 110, an MRI real-time control sequencer 152, and an MRI subsystem 154. The MRI subsystem 154 may include XYZ magnetic gradient coils and associated amplifiers 168, a static Z-axis magnet 169, a digital RF transmitter 162, a digital RF receiver 160, a transmit/receive switch 164, and RF coil(s) 166. The MRI subsystem 154 may be controlled in real time by control sequencer 152 to generate magnetic and radio frequency fields that stimulate magnetic resonance phenomena in a living subject P to be imaged. A contrast-enhanced image of an area of interest A of the subject P may be shown on display 158. The display 158 may be implemented through a variety of output interfaces, including a monitor, printer, or data storage.

The area of interest A corresponds to a region associated with one or more physiological activities in subject P, such as muscular movements. Although not specifically shown in FIG. 1, in some embodiments described herein in accordance with the present disclosure, for example in the descriptions corresponding to FIGS. 3-12, the area of interest can include one or more limbs of a subject, and in particular one or more muscles or groups of muscles in lower limbs.

It should be appreciated that any number and type of computer-based medical imaging systems or components, including various types of commercially available medical imaging systems and components, may be used to practice certain aspects of the present disclosure. Systems as described herein with respect to example embodiments are not intended to be specifically limited to magnetic resonance imaging (MRI) implementations or the particular system shown in FIG. 1.

One or more data acquisition or data collection steps as described herein in accordance with one or more embodiments may include acquiring, collecting, receiving, or otherwise obtaining data such as imaging data corresponding to an area of interest. By way of example, data acquisition or collection may include acquiring data via a data acquisition device, receiving data from an on-site or off-site data acquisition device or from another data collection, storage, or processing device. Similarly, data acquisition or data collection devices of a system in accordance with one or more embodiments of the present disclosure may include any device configured to acquire, collect, or otherwise obtain data, or to receive data from a data acquisition device within the system, an independent data acquisition device located on-site or off-site, or another data collection, storage, or processing device.

FIG. 2 is a computer architecture diagram showing a general computing system capable of implementing aspects of the present disclosure in accordance with one or more embodiments described herein. A computer 200 may be configured to perform one or more functions associated with embodiments illustrated in one or more of FIGS. 3-12. For example, the computer 200 may be configured to perform operations of the method shown in FIG. 3. It should be appreciated that the computer 200 may be implemented within a single computing device or a computing system formed with multiple connected computing devices. The computer 200 may be configured to perform various distributed computing tasks, in which processing and/or storage resources may be distributed among the multiple devices. The data acquisition and display computer 150 and/or operator console 110 of the system shown in FIG. 1 may include one or more systems and components of the computer 200.

As shown, the computer 200 includes a processing unit 202 ("CPU"), a system memory 204, and a system bus 206 that couples the memory 204 to the CPU 202. The computer 200 further includes a mass storage device 212 for storing program modules 214. The program modules 214 may be operable to perform associated with embodiments illustrated in one or more of FIGS. 3-12 discussed below, for example to cause the computer 200 to perform operations of the method shown in FIG. 3. The program modules 214 may include an imaging application 218 for performing data acquisition functions as described herein, for example to receive image data corresponding to magnetic resonance imaging of an area of interest. The computer 200 can include a data store 220 for storing data that may include imaging-related data 222 such as acquired image data, and a modeling data store 224 for storing image modeling data, or other various types of data utilized in practicing aspects of the present disclosure.

The mass storage device 212 is connected to the CPU 202 through a mass storage controller (not shown) connected to the bus 206. The mass storage device 212 and its associated computer-storage media provide non-volatile storage for the computer 200. Although the description of computer-storage media contained herein refers to a mass storage device, such as a hard disk or CD-ROM drive, it should be appreciated by those skilled in the art that computer-storage media can be any available computer storage media that can be accessed by the computer 200.

By way of example and not limitation, computer storage media (also referred to herein as "computer-readable storage medium" or "computer-readable storage media") may include volatile and non-volatile, removable and non-removable media implemented in any method or technology for storage of information such as computer-storage instructions, data structures, program modules, or other data. For example, computer storage media includes, but is not limited to, RAM, ROM, EPROM, EEPROM, flash memory or other solid state memory technology, CD-ROM, digital versatile disks ("DVD"), HD-DVD, BLU-RAY, or other optical storage, magnetic cassettes, magnetic tape, magnetic disk storage or other magnetic storage devices, or any other medium which can be used to store the desired information and which can be accessed by the computer 200. "Computer storage media", "computer-readable storage medium" or "computer-readable storage media" as described herein do not include transitory signals.

According to various embodiments, the computer 200 may operate in a networked environment using connections to other local or remote computers through a network 216 via a network interface unit 210 connected to the bus 206. The network interface unit 210 may facilitate connection of the computing device inputs and outputs to one or more suitable networks and/or connections such as a local area network (LAN), a wide area network (WAN), the Internet, a cellular network, a radio frequency (RF) network, a Bluetooth-enabled network, a Wi-Fi enabled network, a satellite-based network, or other wired and/or wireless networks for communication with external devices and/or systems. The computer 200 may also include an input/output controller 208 for receiving and processing input from any of a number of input devices. Input devices may include one or more of keyboards, mice, stylus, touchscreens, microphones, audio capturing devices, and image/video capturing devices. An end user may utilize the input devices to interact with a user interface, for example a graphical user interface, for managing various functions performed by the computer 200.

The bus 206 may enable the processing unit 202 to read code and/or data to/from the mass storage device 212 or other computer-storage media. The computer-storage media may represent apparatus in the form of storage elements that are implemented using any suitable technology, including but not limited to semiconductors, magnetic materials, optics, or the like. The computer-storage media may represent memory components, whether characterized as RAM, ROM, flash, or other types of technology. The computer storage media may also represent secondary storage, whether implemented as hard drives or otherwise. Hard drive implementations may be characterized as solid state, or may include rotating media storing magnetically-encoded information. The program modules 214, which include the imaging application 218, may include instructions that, when loaded into the processing unit 202 and executed, cause the computer 200 to provide functions associated with one or more embodiments illustrated in FIGS. 3-12. The program modules 214 may also provide various tools or techniques by which the computer 200 may participate within the overall systems or operating environments using the components, flows, and data structures discussed throughout this description.

In general, the program modules 214 may, when loaded into the processing unit 202 and executed, transform the processing unit 202 and the overall computer 200 from a general-purpose computing system into a special-purpose computing system. The processing unit 202 may be constructed from any number of transistors or other discrete circuit elements, which may individually or collectively assume any number of states. More specifically, the processing unit 202 may operate as a finite-state machine, in response to executable instructions contained within the program modules 214. These computer-executable instructions may transform the processing unit 202 by specifying how the processing unit 202 transitions between states, thereby transforming the transistors or other discrete hardware elements constituting the processing unit 202.

Encoding the program modules 214 may also transform the physical structure of the computer-storage media. The specific transformation of physical structure may depend on various factors, in different implementations of this description. Examples of such factors may include, but are not limited to the technology used to implement the computer-storage media, whether the computer storage media are characterized as primary or secondary storage, and the like. For example, if the computer storage media are implemented as semiconductor-based memory, the program modules 214 may transform the physical state of the semiconductor memory, when the software is encoded therein. For example, the program modules 214 may transform the state of transistors, capacitors, or other discrete circuit elements constituting the semiconductor memory.

As another example, the computer storage media may be implemented using magnetic or optical technology. In such implementations, the program modules 214 may transform the physical state of magnetic or optical media, when the software is encoded therein. These transformations may include altering the magnetic characteristics of particular locations within given magnetic media. These transformations may also include altering the physical features or characteristics of particular locations within given optical media, to change the optical characteristics of those locations. Other transformations of physical media are possible without departing from the scope of the present description, with the foregoing examples provided only to facilitate this discussion.

FIG. 3 is a flow diagram illustrating operations of a method 300 for identifying and profiling muscle patterns, according to one embodiment of the present disclosure. As shown, at 302, image data is acquired that is associated with a selected muscle or group of muscles of one or more subjects. At 304, muscle volume of the selected muscle or group of muscles is determined based on the image data. At 306, based on the muscle volume and the height and mass of the one or more subject, a height-mass normalized muscle volume is calculated for the selected muscle or group of muscles. At 308, a deviation is determined of the height-mass normalized muscle volume of the selected muscle or group of muscles from a mean value of muscle volume that is associated with a corresponding reference muscle or reference group of muscles. At 310, a muscle abnormality or absence of muscle abnormality in the selected muscle or group of muscles is identified based on the deviation. At 312, a profile is generated that indicates a pattern of muscle abnormality in the selected muscle or group of muscles.

The method 300 may also include, for a muscle abnormality, identifying an amount or degree of the abnormality. The muscle abnormality may include hypertrophy or atrophy. Hypertrophy may correspond to a normalized muscle volume that is greater than the mean value and atrophy may correspond to a normalized muscle volume that is less than the mean value. Determining the deviation may include calculating an amount of hypertrophy or atrophy of the selected muscle or group of muscles relative to the mean value.

The mean value may be a mean normal value corresponding to a respective normal muscle or group of muscles of one or more reference subjects without a muscle abnormality. The mean value may correspond to a muscle or group of muscles of one or more reference subjects wherein at least one of the one or more subjects has a different amount or degree of muscle abnormality than at least one of the one or more reference subjects.

Acquiring the image data may include acquiring magnetic resonance imaging (MRI) data associated with the selected muscle or group of muscles. The selected muscle or group of muscles may be selected based on the corresponding function the selected muscle or group of muscles performs for the one or more subjects.

The one or more subjects may include a plurality of subjects, with each subject having a muscle abnormality in the respective selected muscle or group of muscles. The method 300 may further include generating a profile indicating a pattern of muscle abnormality across the plurality of subjects. The profile may be generated based on an amount or degree of muscle abnormality corresponding to the selected muscle or group of muscles of each of the plurality of subjects. Generating the profile may include grouping the plurality of subjects based on magnitude of the respective muscle abnormality. Generating the profile may include grouping the plurality of subjects based on particular patterns of muscle abnormality across a predetermined plurality of muscles of each respective one of the plurality of subjects.

Generating the profile may include using non-biased functions for determining the profile. The non-biased functions may include at least one of: hierarchical clustering of the plurality of subjects; principal component analysis; and determining the profile based on a relationship with performance or injury metrics associated with the plurality of subject. The hierarchical clustering may include multi-dimensional hierarchical clustering of the plurality of subjects based on the amount or degree of muscle abnormality across a predetermined plurality of muscles.

The following description provides a further discussion of certain aspects of the present disclosure in accordance with example embodiments. A description of example implementations and results of practicing various aspects of the present disclosure will be presented.

EXAMPLE IMPLEMENTATIONS AND RESULTS

Various aspects of the present disclosure may be still more fully understood from the following description of some example implementations and corresponding results and the images of FIGS. 4-12. Some experimental data are presented herein for purposes of illustration and should not be construed as limiting the scope of the present disclosure in any way or excluding any alternative or additional embodiments.

EXAMPLE 1

A first example (hereinafter referred to as "Example 1") of practicing aspects of the present disclosure will now be described along with corresponding results and with reference to illustrations in FIGS. 4A, 4B, and 5.

*Methods*

Using high resolution MRI, the lower limbs of 20 non-athletes and 20 collegiate athletes were imaged. Using custom image processing software, individual muscles were segmented in 2D axial slices and their volumes computed. In order to investigate overall leg hypertrophy, the relationship between total lower limb muscle volume and the product of height and mass for all subjects was examined. To investigate patterns of hypertrophy across the 35 muscles, the amount of muscle hypertrophy was quantified as the difference from the height-mass normalized volume of each muscle between each athlete and the average normal value, represented as a Z score (standard deviations from normal). ([1]). Using hierarchical clustering, the 35-dimensional Euclidean distance between each athlete's normalized volumes was calculated, and athletes were sorted based on statistically similar hypertrophy patterns. Linkages were based on hypertrophy similarity, and weighted mean hypertrophy patterns of each cluster were computed.

*Results*

FIG. 4A illustrates subject total limb muscle volumes plotted in color-coded regions of hypertrophy, and FIG. 4B illustrates individual limb muscles that are color-coded by the amount of hypertrophy. As shown by FIG. 4A, total lower limb muscle volume of athletes is larger than non-athletes with similar body size. As shown by FIG. 4B, hypertrophy is non-uniform across the lower limb. Muscles do not scale uniformly, with individuals showing different amounts of hypertrophy among their 35 muscles.

FIG. 5 shows a dendogram representation of individual athlete limbs that have been sorted based on 35-D clustering along muscle dimensions. The weighted mean limbs of three primary clusters of similar athletes are shown. Patterns of hypertrophy were similar among athletes with similar athletic performance, as indicated by the three primary clusters. For example, the sartorius is normal in mid-speed athletes (cluster 1), slightly hypertrophied in football and baseball players (cluster 2), and extremely hypertrophied in high-speed athletes (cluster 3). From these results, it may be recognized that athletes may experience non-uniform muscle hypertrophy to optimize specific motion, generating the relationship between hypertrophy patterns and performance.

EXAMPLE 2

A second example (hereinafter referred to as "Example 2") of practicing aspects of the present disclosure will now be described along with corresponding results and with reference to illustrations in FIGS. 6-12.

*Methods*

Twenty-seven competitive collegiate male athletes from varsity basketball (10 athletes), football (5 athletes), baseball (5 athletes), and track and field (7 athletes) teams participated in this study, with the following characteristics (mean $\pm$ st.dev. [range]): age: 20 $\pm$ 1.8 [18-24] years, height: 190.0 $\pm$ 9.8 [175.3-210.8] cm, body mass: 93.0 $\pm$ 16.7 [65.8-138.3] kg (see Table 1 below). All subjects were healthy and competing at the time of this study. Example 1 covers *in vivo* volumes for 35 lower limb muscles in a group of 24 healthy subjects. These subjects discussed in Example 1 were used as non-athlete controls for purposes of Example 2. Characteristics of those subjects are: age: 25.5 $\pm$ 11.1 [12-51], height: 171 $\pm$ 10 [145-188] cm, body mass: 71.8 $\pm$ 14.6 [47.5-107.0] kg.

Athletes' muscle volumes were measured by the same procedure used in the study of Example 1 to measure the control subject. Athletes were scanned on a 3T Siemens (Munich, Germany) Trio MRI Scanner using a 2D multi-slice gradient-echo pulse sequence which utilized a spiral trajectory in k-space for rapid data acquisition ([2]). Scans were completed with the following parameters: TE/TR/$\alpha$: 3.8 ms/ 800 ms/ 90°; FOV: 400 mm $\times$ 400 mm; slice thickness: 5 mm; in plane spatial resolution: 1.1 mm $\times$ 1.1 mm; body receiver coil; and four signal averages. To improve muscle contrast, spectral-spatial excitation pulses were used for fat suppression ([3]). To compensate for spatial variations of the magnetic field, a Chebyshev approximation was applied for semi-automatic off-resonance correction ([4]). Contiguous axial images were obtained from the ankle joint to either the twelfth thoracic vertebra (T12) or the iliac crest (IC). The only muscle to not be fully imaged in subjects whose scans stop at the IC is the psoas; for those subjects, psoas volume is extrapolated to include the region from IC to T12 so that comparisons across subjects are consistent. Scan time varied according to subject height but was approximately 30 minutes per subject.

For each subject, 35 muscles were segmented in both limbs using image processing software written in Matlab (The Mathworks Inc., Natick, MA, USA), where muscle boundaries were outlined to define CSA in each axial slice. Segmentations were completed by 13 trained individuals who were each provided with a detailed slice-by-slice segmentation atlas created from the data set of one of the healthy control subjects. The volume of each muscle was computed from the sum of the CSAs from all axial slices multiplied by the slice thickness. The total muscle volume of a single limb is the sum of the individual volumes of all 35 muscles in that limb. Athletes' dominant limbs were designated as their strongest, and when applicable, non-injured limb.

**Table 1:** Subject information for 27 male collegiate athletes.

| Subject | sport event/ position | Height (cm) | Mass (kg) | Dominant Leg | Right Leg Total Muscle Volume (ml) | Left Leg Total Muscle Volume (ml) | Age |
|---|---|---|---|---|---|---|---|
| **Track & Field** | | | | | | | |
| Track1 | Sprint, High Jump | 193 | 82 | left | 9745 | 9847 | 18 |
| Track2 | Sprint, Hurdles, High and Long Jump | 180 | 75 | right | 9202 | 9625 | 19 |
| Track3 | Long and Triple Jump | 178 | 78 | right | 11021 | 10610 | 18 |
| Track4 | Sprint | 183 | 66 | left | 7972 | 8237 | 19 |
| Track5 | Sprint | 185 | 83 | right | 11016 | 10912 | 18 |
| Track6 | Sprint, Hurdles | 178 | 72 | right | 9880 | 9712 | 20 |
| Track7 | Sprint | 183 | 77 | right | 10872 | 10423 | 18 |
| **Football** | | | | | | | |
| Football1 | Linebacker | 185 | 103 | left | 11354 | 12145 | 22 |
| Football2 | Offensive Guard | 198 | 138 | right | 12241 | 12344 | 22 |
| Football3 | Tight End | 196 | 117 | left | 12411 | 12540 | 23 |
| Football4 | Corner Back | 180 | 80 | right | 10848 | 10868 | 18 |
| Football5 | Kicker | 180 | 79 | right | 9925 | 8983 | 21 |
| Baseball | | | | | | | |
| Baseball1 | Infield/Catcher | 175 | 79 | left | 9892 | 10057 | 19 |
| Baseball2 | Infield/Catcher | 188 | 95 | left | 11232 | 11308 | 22 |
| Baseball3 | Outfield | 178 | 83 | right | 10100 | 9732 | 21 |
| Baseball4 | Outfield/Catcher | 183 | 87 | left | 11082 | 11297 | 20 |
| Baseball5 | Pitcher | 198 | 114 | right | 14311 | 14286 | 22 |
| **Basketball** | | | | | | | |
| Basketball1 | Guard | 196 | 103 | right | 13357 | 13728 | 19 |
| Basketball2 | Forward | 201 | 106 | left | 13575 | 13689 | 21 |
| Basketball3 | Guard | 196 | 94 | right | 11304 | 11691 | 18 |
| Basketball4 | Guard | 198 | 102 | left | 12716 | 12660 | 22 |
| Basketball5 | Forward | 206 | 101 | right | 11547 | 11628 | 19 |
| Basketball6 | Forward | 203 | 103 | right | 14056 | 14341 | 21 |
| Basketball7 | Forward | 198 | 101 | left | 12032 | 11706 | 19 |
| Basketball8 | Guard | 188 | 87 | right | 9891 | 10358 | 19 |
| Basketball9 | Guard | 193 | 93 | right | 10600 | 10625 | 24 |
| Basketball10 | Forward/Center | 211 | 115 | right | 12056 | 12702 | 19 |

In order to compare muscle sizes independently of differences due to body size, muscle volumes were normalized by the product of each subject's height and body mass, which has been shown to be a good predicator of lower limb muscle volumes in healthy people ([1]). This normalization creates a functional metric (muscle volume per height*mass) that can be used to compare muscle capacity per body size between control subjects and athletes in order to quantify hypertrophy. In this comparison, a Z score was computed for each muscle in both limbs of each athlete as follows:

$$Z = \frac{normalized\ volume_{athlete} - mean(normalized\ volume_{control})}{st.dev.(normalized\ volume_{control})}$$

where *normalized volume$_{athlete}$* is the volume per height*mass of a specific muscle in an athlete, *mean(normalized volume$_{control}$)* is the mean volume per height*mass of that muscle in the control group, and *st.dev.(normalized volume$_{control}$)* is the standard deviation of volume per height*mass of that muscle in the control group. Z score is a measure of how many standard deviations an athlete's muscle volume is away from the mean volume of that muscle in the control group, which provides a statistically meaningful measurement of how much an individual athlete's muscle volumes deviate from the muscle volumes of the control subjects. Increasingly positive Z scores represent muscles that are hypertrophic, while negative Z scores represent muscles that are atrophic.

These Z scores were used as the measurement of hypertrophy for each individual muscle in the athletes. A clustering analysis was applied to compare all muscles in all athletes simultaneously. Clustering analyses may generally be used to rearrange the rows and columns a large data matrix based on their similarity in order to reveal significant meaning. To perform the clustering analysis in accordance with this Example 2, all data sets of Z scores were arranged into a matrix where each column corresponded to a unique athlete limb and each row corresponded to a unique muscle (see "A." in FIG. 7). Muscles can be compared by describing each one as a vector, defined by the row values, that exists in multi-dimensional space, in which each dimension is defined by one of the athlete limbs, or columns. Therefore, "athlete space" has as many dimensions as the number of athlete limbs included (in this case 54-dimensional space, with both legs of 27 athletes), and in this space exists as many vectors as there are muscles (in this case 35 vectors).

Muscle vectors can then be clustered by their Euclidian distance in "athlete space", meaning that vectors with the most similar magnitude are grouped (see "B." in FIG. 7). Vectors are linked in pairs with an average linkage to build a hierarchy, meaning that all vectors are compared and the two closest to each other are grouped together; the average of these is taken and then compared with all remaining vectors to identify the new closest pair, of which the average is taken and the process is repeated, each time with one fewer vector being compared, until all the original vectors are linked. These linkages are illustrated in FIG. 7 by a hierarchical dendrogram (see "C."). This same clustering process that was done to the muscles is applied to the athlete limbs. Now the athlete columns (see "D.") define the vectors, and the space they are in has as many dimensions as there are muscles. In this case, "muscle space" is 35 dimensions (see "E."). Then hierarchical clustering of athletes is determined from the Euclidian distance of these vectors in "muscle space". The final result shows the original data matrix with the rows and columns rearranged based on the clustering, depicted by a dendrogram for both the rows and columns (see "F.").

In order to determine the sensitivity of athlete clustering to deviations in hypertrophy of particular muscles, principal component analysis (PCA) of the "muscle space" was performed. PCA redefines the data space by creating new dimensional vectors, called principal components, from linear combinations of the original dimensional vectors (in this case muscle vectors), in order to more of the total variance in fewer dimensions. This method is used to take complicated multi-dimensional data and reduce the number of dimensions needed to display a significant portion of the original data. PCA is executed by defining a vector, that is a linear combination of the original vectors, that captures the highest percentage of the data's variance, then finding another vector, orthogonal to the first, that captures the next highest percent of the variance; this continues until there are as many dimensions as the original data space but each of these components captures increasingly less of the data space variance. Each of these components makes up some percentage of the overall variance in the (athlete) data, and is defined by a vector of coefficients describing each of the original (muscle) vectors' contributions to it. Each component's coefficients are weighted by the amount of variance that vector describes, and then the absolute values of the weighted coefficients for each muscle are summed for all components, to determine how individual muscles influence the clustering of athletes.

*Results*

A previous study ([1]) has shown that the product of body mass and height can reliably predict total lower limb muscle volume in healthy adults and that the volume of individual muscles scales linearly with that total volume. The subjects from this previous study ([1]) were used as the control group to compare to the athletes for Example 2. When total muscle volume in the dominant limb of athletes is plotted with their height and mass, most fall above the healthy controls, indicating that athletes have more muscle volume for their body size than predicted by the scaling relationship in healthy people. All athletes except four basketball players and two football players have more than one standard deviation greater than the mean of the control's total muscle volume per height*mass. Of these, two football players, three baseball players, and four track and field athletes had more than 2.5 standard deviations more volume per height*mass. Although athletes typically have more muscle volume than controls, increased volume is not consistently scaled up with body size.

Not only is total muscle volume inconsistently different between athletes and controls, individual muscle volumes in athletes are not uniformly different from the mean of control muscles. The mean normalized volume of 24 muscles in the athletes falls within one standard deviation of the mean volume of controls, and of these, distal muscles tend to be below the mean while more proximal muscles are above it. The mean of 11 muscles in the athletes have a Z score greater than 1, including the gluteus maximus, sartorius, semitendinosus, and all four quadriceps. For individual muscles, not only is the mean of the athlete population non-uniformly different from the mean of controls, there is also a large amount of variation between athletes for each muscle. For example, the gluteus maximus in the athletes on average has a Z score close to 2.5, but some athletes have a Z score greater than 5 and others below -1 with variation in between. Therefore, athlete muscles not only display non-uniform hypertrophy compared to the controls, but also display that hypertrophy varies considerably between individual athletes.

Hypertrophy in athletes cannot be fully characterized by reducing athletes to the sum of their muscle volumes or by describing muscles by the average volume of the athlete population, so additional analysis is required to consider individual muscles and athletes simultaneously. Athletes have unique patterns of hypertrophy in their lower limb muscles, quantified by the Z score compared to control muscles, which identify individual phenotypes. Clustering analysis enables comparison of these phenotypes and groups athletes with others that have similar patterns of hypertrophy. For the 27 athletes in the study, both the dominant and non-dominant leg is included in the clustering analysis. For all but three athletes, their dominant and non-dominant limbs group with each other before any other athletes, meaning that the phenotype of an individual's two legs have more in common than the phenotypes of two different athletes. Of the three athletes whose contralateral limbs do not cluster, one had an ACL repair surgery in one knee, one had a significant unilateral hamstring injury, and one is a football kicker who asymmetrically trains his dominant and non-dominant legs. In the same way as athletes, individual muscles are compared and grouped based on their pattern of hypertrophy across the population of athletes.

Because most athletes' non-dominant leg clusters with their dominant leg, the phenotype of the dominant leg alone can be used to compare between athletes. As illustrated in FIG. 11, when clustered, athletes whose phenotypes are characterized a large amount of hypertrophy group to the left, and athletes whose phenotypes exhibit limited hypertrophy and increased atrophy group to the right. While many athletes group with athletes who compete in their own sport, often they group with athletes who compete in different sports, and different positions or events in that sport. For instance, the two athletes grouped to the far left both compete in track and field but one does sprint and hurdle events and the other does the long and triple jump. To the far right, a football offensive guard clusters with a basketball player who plays forward and center. Athletes in different sports and positions group together, indicating that individual phenotypes are not solely influenced by the sport an athlete competes in.

Clustered muscles have the most similar hypertrophy across the athlete population, which sometimes are also muscles with similar function in the limb. For example, the top cluster shown in FIG. 10 includes the rectus femoris and the vastus medialis and lateralis, which are all knee extensors. But this group also includes the semitendinosus, gluteus maximus, and sartorius, a knee flexor, hip extensor, and hip flexor, respectively. While the clustering shows which muscles are similar to each other, it does not explicitly reveal which muscles are most significantly affecting how the athletes are grouped. Examining the principal components of the data provides insight into how sensitive the clustering is to hypertrophy in particular muscles. The principal components are new vectors that redefine the data space, each capturing increasing less of the overall variance. Each muscle has a coefficient characterizing its contribution to each component. When these coefficients are weighted by the amount of overall variance of the components, summing all the weighted coefficients for a single muscle characterizes that muscle's contribution to the overall variance of the data determining the clustering. Athlete clustering was more sensitive to hypertrophy in the muscles with higher weighted sums (see FIG. 12). Weighted sums of muscles in the quadriceps and hamstrings tend to be higher, while in hip rotator, plantarflexor, and dorsiflexor muscles, sums tend to be lower, indicating that these knee crossing muscles have a more significant affect in determining athlete phenotypes than these hip and ankle muscles.

CONCLUSION

The specific configurations, choice of materials and the size and shape of various elements can be varied according to particular design specifications or constraints requiring a system or method constructed according to the principles of the present disclosure. Such changes are intended to be embraced within the scope of the present disclosure. The presently disclosed embodiments, therefore, are considered in all respects to be illustrative and not restrictive. The scope of the invention is defined by the appended claims.

**EP 3 164 069 B1**

REFERENCE LIST

[1] Handsfield, G. C., et al., Relationships of 35 lower limb muscles to height and body mass quantified using MRI. Journal of Biomechanics (2013).
[2] Meyer, C. H., et al., Fast spiral coronary artery imaging. Magnetic Resonance in Medicine (1992).
[3] Meyer, C. H., et al., Simultaneous spatial and spectral selective excitation. Magnetic Resonance in Medicine 15: 287-304 (1990).
[4] Chen, W., et al., Fast conjugate phase image reconstruction based on a Chebyshev approximation to correct for B0 field inhomogeneity and concomitant gradients. Magnetic Resonance in Medicine (2008).

**Claims**

1. A method, comprising:

    acquiring (302) image data associated with a selected muscle or group of muscles of one or more subjects by receiving data from a data acquisition device or a data collection, storage or processing device;
    determining (304), based on the image data, muscle volume of the selected muscle or group of muscles;
    calculating (306), based on the muscle volume and the height and mass of the one or more subjects, a height-mass normalized muscle volume for the selected muscle or group of muscles;
    determining (308) a deviation of the height-mass normalized muscle volume of the selected muscle or group of muscles from a mean value of muscle volume associated with a corresponding reference muscle or reference group of muscles; and
    identifying (312), based on the deviation, a muscle abnormality or absence of a muscle abnormality in the selected muscle or group of muscles,
    wherein the one or more subjects comprise a plurality of subjects, each subject having a muscle abnormality in the respective selected muscle or group of muscles, and wherein the method further comprises generating a profile indicating a pattern of muscle abnormality across the plurality of subjects,
    wherein generating the profile comprises grouping the plurality of subjects based on particular patterns of muscle abnormality across a predetermined plurality of muscles of each respective one of the plurality of subjects,
    wherein generating the profile comprises using non-biased functions for determining the profile, the non-biased functions including hierarchical clustering of the plurality of subjects, and
    wherein the hierarchical clustering comprises multi-dimensional hierarchical clustering of the plurality of subjects based on the amount or degree of muscle abnormality across a predetermined plurality of muscles.

2. The method of claim 1, further comprising, for a muscle abnormality, identifying an amount or degree of the abnormality, or
    wherein the mean value is a mean normal value corresponding to a respective normal muscle or group of muscles of one or more reference subjects without a muscle abnormality, or
    wherein the mean value corresponds to a muscle or group of muscles of one or more reference subjects, and wherein at least one of the one or more subjects has a different amount or degree of muscle abnormality than at least one of the one or more reference subjects, or
    wherein acquiring the image data comprises acquiring magnetic resonance imaging (MRI) data associated with the selected muscle or group of muscles, or
    wherein the selected muscle or group of muscles is selected based on the corresponding function the selected muscle or group of muscles performs for the one or more subjects.

3. The method of claim 1, wherein the muscle abnormality comprises hypertrophy or atrophy, and optionally:

    wherein hypertrophy corresponds to a normalized muscle volume that is greater than the mean value and atrophy corresponds to a normalized muscle volume that is less than the mean value, or
    wherein determining the deviation comprises calculating an amount of hypertrophy or atrophy of the selected muscle or group of muscles relative to the mean value.

4. The method of claim 1, wherein the profile is generated based on an amount or degree of muscle abnormality corresponding to the selected muscle or group of muscles of each of the plurality of subjects, or

wherein generating the profile comprises grouping the plurality of subjects based on magnitude of the respective muscle abnormality.

5. The method of claim 1, wherein the non-biased functions further includes at least one of:

principal component analysis; and
determining the profile based on a relationship with performance or injury metrics associated with the plurality of subjects.

6. A system, comprising:

a data acquisition device configured to acquire image data associated with a selected muscle or group of muscles of one or more subjects; and
a processing device configured to perform functions that include:

determining (304), based on the image data, muscle volume of the selected muscle or group of muscles;
calculating (306), based on the muscle volume and the height and mass of the one or more subjects, a height-mass normalized muscle volume for the selected muscle or group of muscles;
determining (308) a deviation of the height-mass normalized muscle volume of the selected muscle or group of muscles from a mean value of muscle volume associated with a corresponding reference muscle or reference group of muscles; and
identifying (310), based on the deviation, a muscle abnormality or absence of a muscle abnormality in the selected muscle or group of muscles,

wherein the one or more subjects comprise a plurality of subjects, each subject having a muscle abnormality in the respective selected muscle or group of muscles, and wherein the processing device is further configured to generate a profile indicating a pattern of muscle abnormality across the plurality of subjects,
wherein generating the profile comprises grouping the plurality of subjects based on particular patterns of muscle abnormality across a predetermined plurality of muscles of each respective one of the plurality of subjects,
wherein generating the profile comprises using non-biased functions for determining the profile, the non-biased functions including hierarchical clustering of the plurality of subjects, and
wherein the hierarchical clustering comprises multi-dimensional hierarchical clustering of the plurality of subjects based on the amount or degree of muscle abnormality across a predetermined plurality of muscles.

7. The system of claim 6, wherein the processing device is further configured to identify, for a muscle abnormality, an amount or degree of the abnormality, or
wherein the mean value is a mean normal value corresponding to a respective normal muscle or group of muscles of one or more reference subjects without a muscle abnormality, or
wherein the mean value corresponds to a muscle or group of muscles of one or more reference subjects, and wherein at least one of the one or more subjects has a different amount or degree of muscle abnormality than at least one of the one or more reference subjects, or
wherein acquiring the image data comprises acquiring magnetic resonance imaging (MRI) data associated with the selected muscle or group of muscles, or
wherein the selected muscle or group of muscles is selected based on the corresponding function the selected muscle or group of muscles performs for the one or more subjects.

8. The system of claim 6, wherein the muscle abnormality comprises hypertrophy or atrophy, and optionally:

wherein hypertrophy corresponds to a normalized muscle volume that is greater than the mean value and atrophy corresponds to a normalized muscle volume that is less than the mean value, or
wherein determining the deviation comprises calculating an amount of hypertrophy or atrophy of the selected muscle or group of muscles relative to the mean value.

9. The system of claim 6, wherein the profile is generated based on an amount or degree of muscle abnormality corresponding to the selected muscle or group of muscles of each of the plurality of subjects, or
wherein generating the profile comprises grouping the plurality of subjects based on magnitude of the respective muscle abnormality.

**10.** The system of claim 6, wherein the non-biased function further includes at least one of:

> principal component analysis; and
> determining the profile based on a relationship with performance or injury metrics associated with the plurality of subjects.

**11.** A non-transitory computer-readable medium storing instructions that, when executed by one or more processors, cause a computing device to perform a method that comprises:

> acquiring (302) image data associated with a selected muscle or group of muscles of one or more subjects, by receiving data from a data acquisition device or a data collection, storage or processing device,
> determining (304), based on the image data, muscle volume of the selected muscle or group of muscles;
> calculating (306), based on the muscle volume and the height and mass of the one or more subjects, a height-mass normalized muscle volume for the selected muscle or group of muscles;
> determining (308) a deviation of the height-mass normalized muscle volume of the selected muscle or group of muscles from a mean value of muscle volume associated with a corresponding reference muscle or reference group of muscles; and
> identifying (310), based on the deviation, a muscle abnormality or absence of a muscle abnormality in the selected muscle or group of muscles,
> wherein the one or more subjects comprise a plurality of subjects, each subject having a muscle abnormality in the respective selected muscle or group of muscles, and wherein the method performed by the computing device further comprises generating a profile indicating a pattern of muscle abnormality across the plurality of subjects,
> wherein generating the profile comprises grouping the plurality of subjects based on particular patterns of muscle abnormality across a predetermined plurality of muscles of each respective one of the plurality of subjects,
> wherein generating the profile comprises using non-biased functions for determining the profile, the non-biased functions including hierarchical clustering of the plurality of subjects, and
> wherein the hierarchical clustering comprises multi-dimensional hierarchical clustering of the plurality of subjects based on the amount or degree of muscle abnormality across a predetermined plurality of muscles.

**12.** The non-transitory computer-readable medium of claim 11, wherein the method performed by the computing device further comprises identifying, for a muscle abnormality, an amount or degree of the abnormality, or
wherein the mean value is a mean normal value corresponding to a respective normal muscle or group of muscles of one or more reference subjects without a muscle abnormality, or
wherein the mean value corresponds to a muscle or group of muscles of one or more reference subjects, and wherein at least one of the one or more subjects has a different amount or degree of muscle abnormality than at least one of the one or more reference subjects, or
wherein acquiring the image data comprises acquiring magnetic resonance imaging (MRI) data associated with the selected muscle or group of muscles, or
wherein the selected muscle or group of muscles is selected based on the corresponding function the selected muscle or group of muscles performs for the one or more subjects.

**13.** The non-transitory computer-readable medium of claim 11, wherein the muscle abnormality comprises hypertrophy or atrophy, and optionally:

> wherein hypertrophy corresponds to a normalized muscle volume that is greater than the mean value and atrophy corresponds to a normalized muscle volume that is less than the mean value, or
> wherein determining the deviation comprises calculating an amount of hypertrophy or atrophy of the selected muscle or group of muscles relative to the mean value.

**14.** The non-transitory computer-readable medium of claim 11, wherein the profile is generated based on an amount or degree of muscle abnormality corresponding to the selected muscle or group of muscles of each of the plurality of subjects, or
wherein generating the profile comprises grouping the plurality of subjects based on magnitude of the respective muscle abnormality.

**15.** The non-transitory computer-readable medium of claim 11, wherein the non-biased functions further includes at least one of:

principal component analysis; and
determining the profile based on a relationship with performance or injury metrics associated with the plurality
of subjects.

**Patentansprüche**

1. Verfahren, umfassend:

Erfassen (302) von Bilddaten, die einem ausgewählten Muskel oder einer Gruppe von Muskeln eines oder mehrerer Subjekte zugeordnet sind, durch ein Empfangen von Daten von einer Datenerfassungsvorrichtung oder einer Datenerfassungs-, -speicherungs- oder - verarbeitungsvorrichtung;
Bestimmen (304) auf Grundlage der Bilddaten des Muskelvolumens des ausgewählten Muskels oder der ausgewählten Gruppe von Muskeln;
Berechnen (306) auf Grundlage des Muskelvolumens und der Höhe und Masse des einen oder der mehreren Subjekte eines normalisierten Höhe-Masse-Muskelvolumens für den ausgewählten Muskel oder die ausgewählte Gruppe von Muskeln;
Bestimmen (308) einer Abweichung des normalisierten Höhe-Masse-Muskelvolumens des ausgewählten Muskels oder der ausgewählten Gruppe von Muskeln von einem Mittelwert des Muskelvolumens, der einem entsprechenden Referenzmuskel oder einer entsprechenden Referenzgruppe von Muskeln zugeordnet ist; und
Identifizieren (312) auf Grundlage der Abweichung einer Muskelanomalie oder des Fehlens einer Muskelanomalie in dem ausgewählten Muskel oder einer ausgewählten Gruppe von Muskeln,
wobei das eine oder die mehreren Subjekte mehrere Subjekte umfassen, wobei jedes Subjekt eine Muskelanomalie in dem jeweils ausgewählten Muskel oder der jeweils ausgewählten Gruppe von Muskeln aufweist,
und wobei das Verfahren ferner das Erzeugen eines Profils umfasst, das ein Muster einer Muskelanomalie über die mehreren Subjekte anzeigt,
wobei das Erzeugen des Profils das Gruppieren der mehreren Subjekte auf Grundlage von bestimmten Mustern von Muskelanomalien über vorbestimmte mehrere Muskeln jedes jeweiligen der mehreren Subjekte umfasst,
wobei das Erzeugen des Profils das Verwenden von unverzerrten Funktionen zum Bestimmen des Profils umfasst, wobei die unverzerrten Funktionen hierarchische Clusterbildung der mehreren Subjekte enthält, und wobei die hierarchische Clusterbildung eine mehrdimensionale hierarchische Clusterbildung der mehreren Subjekte auf Grundlage der Menge oder des Grads der Muskelanomalie über vorbestimmte mehrere Muskel umfasst.

2. Verfahren nach Anspruch 1, ferner umfassend für eine Muskelanomalie das Identifizieren einer Menge oder eines Grads der Anomalie oder
wobei der Mittelwert ein mittlerer Normalwert ist, der einem jeweiligen normalen Muskel oder einer Gruppe von Muskeln eines oder mehrerer Referenzsubjekte ohne eine Muskelanomalie entspricht oder
wobei der Mittelwert einem Muskel oder einer Gruppe von Muskeln eines oder mehrerer Referenzsubjekte entspricht und wobei das eine und/oder die mehreren Subjekte eine andere Menge oder einen anderen Grad an Muskelanomalie aufweist als das eine und/oder die mehreren Referenzsubjekte, oder
wobei das Erfassen der Bilddaten das Erfassen von Magnetresonanztomographie- (MRT-) Daten umfasst, die dem ausgewählten Muskel oder der ausgewählten Gruppe von Muskeln zugeordnet sind, oder
wobei der ausgewählte Muskel oder die ausgewählte Gruppe von Muskeln auf Grundlage der entsprechenden Funktion ausgewählt ist, die der ausgewählte Muskel oder die Gruppe von Muskeln für das eine oder die mehreren Subjekte durchführt.

3. Verfahren nach Anspruch 1, wobei die Muskelanomalie Hypertrophie oder Atrophie umfasst und optional:

wobei Hypertrophie einem normalisierten Muskelvolumen entspricht, das größer als der Mittelwert ist, und Atrophie einem normalisierten Muskelvolumen entspricht, das kleiner als der Mittelwert ist, oder
wobei das Bestimmen der Abweichung das Berechnen einer Menge an Hypertrophie oder Atrophie des ausgewählten Muskels oder der ausgewählten Gruppe von Muskeln relativ zu dem Mittelwert umfasst.

4. Verfahren nach Anspruch 1, wobei das Profil auf Grundlage einer Menge oder eines Grads von Muskelanomalie erzeugt wird, die dem ausgewählten Muskel oder der ausgewählten Gruppe von Muskeln jedes der mehreren Subjekte entspricht,
oder wobei das Erzeugen des Profils das Gruppieren der mehreren Subjekte auf Grundlage der Größe der jeweiligen Muskelanomalie umfasst.

5. Verfahren nach Anspruch 1, wobei die unverzerrte Funktionen ferner enthält:

Hauptkomponentenanalyse; und/oder
Bestimmen des Profils auf Grundlage einer Beziehung zu Leistungs- oder Verletzungsmetriken, die mit den mehreren Subjekten verbunden sind.

6. System, umfassend:

eine Datenerfassungsvorrichtung, die konfiguriert ist, um Bilddaten zu erfassen, die einem ausgewählten Muskel oder einer Gruppe von Muskeln eines oder mehrerer Subjekte zugeordnet sind; und
eine Verarbeitungsvorrichtung, die konfiguriert ist, um Funktionen auszuführen, die enthalten:

Bestimmen (304) auf Grundlage der Bilddaten des Muskelvolumens des ausgewählten Muskels oder der ausgewählten Gruppe von Muskeln;
Berechnen (306) auf Grundlage des Muskelvolumens und der Höhe und Masse des einen oder der mehreren Subjekte eines normalisierten Höhe-Masse-Muskelvolumens für den ausgewählten Muskel oder die ausgewählte Gruppe von Muskeln;
Bestimmen (308) einer Abweichung des normalisierten Höhe-Masse-Muskelvolumens des ausgewählten Muskels oder der ausgewählten Gruppe von Muskeln von einem Mittelwert des Muskelvolumens, der einem entsprechenden Referenzmuskel oder einer entsprechenden Referenzgruppe von Muskeln zugeordnet ist; und
Identifizieren (310) auf Grundlage der Abweichung einer Muskelanomalie oder des Fehlens einer Muskelanomalie in dem ausgewählten Muskel oder einer ausgewählten Gruppe von Muskeln,

wobei das eine oder die mehreren Subjekte mehrere Subjekte umfassen, wobei jedes Subjekt eine Muskelanomalie in dem jeweils ausgewählten Muskel oder der jeweils ausgewählten Gruppe von Muskeln aufweist, und wobei die Verarbeitungsvorrichtung ferner konfiguriert ist, um ein Profil zu erzeugen, das ein Muster einer Muskelanomalie über die mehreren Subjekte anzeigt,
wobei das Erzeugen des Profils das Gruppieren der mehreren Subjekte auf Grundlage von bestimmten Mustern von Muskelanomalien über vorbestimmte mehrere Muskeln jedes jeweiligen der mehreren Subjekte umfasst,
wobei das Erzeugen des Profils das Verwenden von unverzerrten Funktionen zum Bestimmen des Profils umfasst, wobei die unverzerrten Funktionen hierarchische Clusterbildung der mehreren Subjekte enthält, und wobei die hierarchische Clusterbildung eine mehrdimensionale hierarchische Clusterbildung der mehreren Subjekte auf Grundlage der Menge oder des Grads der Muskelanomalie über vorbestimmte mehrere Muskel umfasst.

7. System nach Anspruch 6, wobei die Verarbeitungsvorrichtung ferner konfiguriert ist, um für eine Muskelanomalie eine Menge oder einen Grad der Anomalie zu identifizieren, oder
wobei der Mittelwert ein mittlerer Normalwert ist, der einem jeweiligen normalen Muskel oder einer Gruppe von Muskeln eines oder mehrerer Referenzsubjekte ohne eine Muskelanomalie entspricht oder
wobei der Mittelwert einem Muskel oder einer Gruppe von Muskeln eines oder mehrerer Referenzsubjekte entspricht und wobei das eine und/oder die mehreren Subjekte eine andere Menge oder einen anderen Grad an Muskelanomalie aufweist als das eine und/oder die mehreren Referenzsubjekte, oder
wobei das Erfassen der Bilddaten das Erfassen von Magnetresonanztomographie- (MRT-) Daten umfasst, die dem ausgewählten Muskel oder der ausgewählten Gruppe von Muskeln zugeordnet sind, oder
wobei der ausgewählte Muskel oder die ausgewählte Gruppe von Muskeln auf Grundlage der entsprechenden Funktion ausgewählt ist, die der ausgewählte Muskel oder die Gruppe von Muskeln für das eine oder die mehreren Subjekte durchführt.

8. System nach Anspruch 6, wobei die Muskelanomalie Hypertrophie oder Atrophie umfasst und optional:

wobei Hypertrophie einem normalisierten Muskelvolumen entspricht, das größer als der Mittelwert ist, und Atrophie einem normalisierten Muskelvolumen entspricht, das kleiner als der Mittelwert ist, oder
wobei das Bestimmen der Abweichung das Berechnen einer Menge an Hypertrophie oder Atrophie des ausgewählten Muskels oder der ausgewählten Gruppe von Muskeln relativ zu dem Mittelwert umfasst.

9. System nach Anspruch 6, wobei das Profil auf Grundlage einer Menge oder eines Grads von Muskelanomalie erzeugt wird, die dem ausgewählten Muskel oder der ausgewählten Gruppe von Muskeln jedes der mehreren Subjekte entspricht, oder
wobei das Erzeugen des Profils das Gruppieren der mehreren Subjekte auf Grundlage der Größe der jeweiligen Muskelanomalie umfasst.

10. System nach Anspruch 6, wobei die unverzerrte Funktion ferner enthält:

    Hauptkomponentenanalyse; und/oder
    Bestimmen des Profils auf Grundlage einer Beziehung zu Leistungs- oder Verletzungsmetriken, die mit den mehreren Subjekten verbunden sind.

11. Nichtflüchtiges computerlesbares Medium, das Anweisungen speichert, die, wenn sie von einem oder mehreren Prozessoren ausgeführt werden, eine Rechenvorrichtung veranlassen, ein Verfahren auszuführen, das umfasst:

    Erfassen (302) von Bilddaten, die einem ausgewählten Muskel oder einer Gruppe von Muskeln eines oder mehrerer Subjekte zugeordnet sind, durch das Empfangen von Daten von einer Datenerfassungsvorrichtung oder einer Datenerfassungs-, -speicherungs- oder - verarbeitungsvorrichtung,
    Bestimmen (304) auf Grundlage der Bilddaten des Muskelvolumens des ausgewählten Muskels oder der Gruppe von Muskeln;
    Berechnen (306) auf Grundlage des Muskelvolumens und der Höhe und Masse des einen oder der mehreren Subjekte eines normalisierten Höhe-Masse-Muskelvolumens für den ausgewählten Muskel oder die ausgewählte Gruppe von Muskeln;
    Bestimmen (308) einer Abweichung des normalisierten Höhe-Masse-Muskelvolumens des ausgewählten Muskels oder der ausgewählten Gruppe von Muskeln von einem Mittelwert des Muskelvolumens, der einem entsprechenden Referenzmuskel oder einer entsprechenden Referenzgruppe von Muskeln zugeordnet ist; und
    Identifizieren (310) auf Grundlage der Abweichung einer Muskelanomalie oder des Fehlens einer Muskelanomalie in dem ausgewählten Muskel oder einer ausgewählten Gruppe von Muskeln,
    wobei das eine oder die mehreren Subjekte mehrere Subjekte umfassen, wobei jedes Subjekt eine Muskelanomalie in dem jeweils ausgewählten Muskel oder der jeweils ausgewählten Gruppe von Muskeln aufweist, und wobei das Verfahren, das durch die Rechenvorrichtung durchgeführt wird, ferner das Erzeugen eines Profils umfasst, das ein Muster einer Muskelanomalie über die mehreren Subjekte anzeigt,
    wobei das Erzeugen des Profils das Gruppieren der mehreren Subjekte auf Grundlage von bestimmten Mustern von Muskelanomalien über vorbestimmte mehrere Muskeln jedes jeweiligen der mehreren Subjekte umfasst,
    wobei das Erzeugen des Profils das Verwenden von unverzerrten Funktionen zum Bestimmen des Profils umfasst, wobei die unverzerrten Funktionen hierarchische Clusterbildung der mehreren Subjekte enthält, und wobei die hierarchische Clusterbildung eine mehrdimensionale hierarchische Clusterbildung der mehreren Subjekte auf Grundlage der Menge oder des Grads der Muskelanomalie über vorbestimmte mehrere Muskel umfasst.

12. Nichtflüchtiges, computerlesbares Medium nach Anspruch 11, wobei das Verfahren, das durch die Rechenvorrichtung ausgeführt wird, ferner das Identifizieren für eine Muskelanomalie einer Menge oder eines Grads der Anomalie umfasst, oder
wobei der Mittelwert ein mittlerer Normalwert ist, der einem jeweiligen normalen Muskel oder einer Gruppe von Muskeln eines oder mehrerer Referenzsubjekte ohne eine Muskelanomalie entspricht oder
wobei der Mittelwert einem Muskel oder einer Gruppe von Muskeln eines oder mehrerer Referenzsubjekte entspricht und wobei das eine und/oder die mehreren Subjekte eine andere Menge oder einen anderen Grad an Muskelanomalie aufweist als das eine und/oder die mehreren Referenzsubjekte, oder
wobei das Erfassen der Bilddaten das Erfassen von Magnetresonanztomographie- (MRT-) Daten umfasst, die dem ausgewählten Muskel oder der ausgewählten Gruppe von Muskeln zugeordnet sind, oder
wobei der ausgewählte Muskel oder die ausgewählte Gruppe von Muskeln auf Grundlage der entsprechenden Funktion ausgewählt ist, die der ausgewählte Muskel oder die Gruppe von Muskeln für das eine oder die mehreren Subjekte durchführt.

13. Nichtflüchtiges computerlesbares Medium nach Anspruch 11, wobei die Muskelanomalie Hypertrophie oder Atrophie umfasst und optional:

    wobei Hypertrophie einem normalisierten Muskelvolumen entspricht, das größer als der Mittelwert ist, und Atrophie einem normalisierten Muskelvolumen entspricht, das kleiner als der Mittelwert ist, oder
wobei das Bestimmen der Abweichung das Berechnen einer Menge an Hypertrophie oder Atrophie des ausgewählten Muskels oder der ausgewählten Gruppe von Muskeln relativ zu dem Mittelwert umfasst.

**14.** Nichtflüchtiges computerlesbares Medium nach Anspruch 11, wobei das Profil auf Grundlage einer Menge oder eines Grads von Muskelanomalie erzeugt wird, die dem ausgewählten Muskel oder der ausgewählten Gruppe von Muskeln jedes der mehreren Subjekte entspricht, oder
wobei das Erzeugen des Profils das Gruppieren der mehreren Subjekte auf Grundlage der Größe der jeweiligen Muskelanomalie umfasst.

**15.** Nichtflüchtiges computerlesbares Medium nach Anspruch 11, wobei die unverzerrten Funktionen ferner enthalten:

Hauptkomponentenanalyse; und/oder
Bestimmen des Profils auf Grundlage einer Beziehung zu Leistungs- oder Verletzungsmetriken, die mit den mehreren Subjekten verbunden sind.

**Revendications**

1. Procédé comprenant :

l'acquisition (302) de données d'image associées à un muscle ou groupe de muscles sélectionné d'un ou plusieurs sujets en recevant des données provenant d'un dispositif d'acquisition de données ou d'un dispositif de collecte, de stockage ou de traitement de données ;
la détermination (304), sur la base des données d'image, du volume musculaire du muscle ou groupe de muscles sélectionné ;
le calcul (306), sur la base du volume musculaire et de la taille et du poids desdits un ou plusieurs sujets, d'un volume musculaire normalisé en hauteur-poids pour le muscle ou groupe de muscles sélectionné ;
la détermination (308) d'un écart du volume musculaire normalisé en hauteur-poids du muscle ou groupe de muscles sélectionné à partir d'une valeur moyenne du volume musculaire associée à un muscle de référence ou groupe de muscles de référence correspondant ; et
l'identification (312), sur la base de l'écart, d'une anomalie musculaire ou de l'absence d'une anomalie musculaire dans le muscle ou groupe de muscles sélectionné, lesdits un ou plusieurs sujets comprenant une pluralité de sujets, chaque sujet possédant une anomalie musculaire dans le muscle ou groupe de muscles sélectionné respectif, et ledit procédé comprenant en outre la génération d'un profil indiquant un modèle d'anomalie musculaire parmi la pluralité de sujets, ladite génération du profil comprenant le regroupement de la pluralité de sujets sur la base de modèles particuliers d'anomalies musculaires parmi une pluralité prédéfinie de muscles de chaque sujet respectif de la pluralité de sujets, ladite génération du profil comprenant l'utilisation de fonctions non biaisées pour déterminer le profil, les fonctions non biaisées comprenant le regroupement hiérarchique de la pluralité de sujets, et ledit regroupement hiérarchique comprenant le regroupement hiérarchique multidimensionnel de la pluralité de sujets en fonction de la quantité ou du degré d'anomalie musculaire parmi une pluralité prédéfinie de muscles.

2. Procédé selon la revendication 1, comprenant en outre, pour une anomalie musculaire, l'identification d'une quantité ou d'un degré de l'anomalie, ou ladite valeur moyenne étant une valeur normale moyenne correspondant à un muscle ou groupe de muscles normal respectif d'un ou plusieurs sujets de référence sans anomalie musculaire, ou ladite valeur moyenne correspondant à un muscle ou groupe de muscles d'un ou plusieurs sujets de référence, et au moins l'un desdits un ou plusieurs sujets présentant une quantité ou un degré d'anomalie musculaire différent d'au moins l'un desdits un ou plusieurs sujets de référence, ou ladite acquisition des données d'image comprenant l'acquisition de données d'imagerie par résonance magnétique (IRM) associées au muscle ou groupe de muscles sélectionné, ou ledit muscle ou groupe de muscles sélectionné étant sélectionné sur la base de la fonction correspondante avec laquelle le muscle ou groupe de muscles sélectionné opère pour lesdits un ou plusieurs sujets.

3. Procédé selon la revendication 1, ladite anomalie musculaire comprenant une hypertrophie ou une atrophie, et éventuellement :
ladite hypertrophie correspondant à un volume musculaire normalisé qui est supérieur à la valeur moyenne et ladite atrophie correspondant à un volume musculaire normalisé qui est inférieur à la valeur moyenne, ou ladite détermination de l'écart comprenant le calcul d'une quantité d'hypertrophie ou d'atrophie du muscle ou groupe de muscles sélectionné par rapport à la valeur moyenne.

4. Procédé selon la revendication 1, ledit profil est généré sur la base d'une quantité ou d'un degré d'anomalie musculaire correspondant au muscle ou groupe de muscles sélectionné de chacun de la pluralité de sujets, ou ladite génération du profil comprenant le regroupement de la pluralité de sujets en fonction de l'ampleur de l'anomalie musculaire respective.

5. Procédé selon la revendication 1, lesdites fonctions non biaisées comprenant en outre au moins l'une parmi :

l'analyse des composants principaux ; et
la détermination du profil sur la base d'une relation avec des paramètres de performance ou de blessure associes à la pluralité de sujets.

6. Système comprenant :

un dispositif d'acquisition de données configuré pour acquérir des données d'image associées à un muscle ou groupe de muscles sélectionné d'un ou plusieurs sujets ; et
un dispositif de traitement configuré pour exécuter des fonctions qui incluent :

la détermination (304), sur la base des données d'image, du volume musculaire du muscle ou groupe de muscles sélectionné ;
le calcul (306), sur la base du volume musculaire et de la taille et du poids desdits un ou plusieurs sujets, d'un volume musculaire normalisé en hauteur-poids pour le muscle ou groupe de muscles sélectionné ;
la détermination (308) d'un écart du volume musculaire normalisé en hauteur-poids du muscle ou groupe de muscles sélectionné à partir d'une valeur moyenne du volume musculaire associée à un muscle de référence ou groupe de muscles de référence correspondant ; et
l'identification (310), sur la base de l'écart, d'une anomalie musculaire ou de l'absence d'une anomalie musculaire dans le muscle ou groupe de muscles sélectionné, lesdits un ou plusieurs sujets comprenant une pluralité de sujets, chaque sujet possédant une anomalie musculaire dans le muscle ou groupe de muscles sélectionné respectif, et ledit dispositif de traitement étant en outre configuré pour générer un profil indiquant un modèle d'anomalie musculaire parmi la pluralité de sujets, ladite génération du profil comprenant le regroupement de la pluralité de sujets sur la base de modèles particuliers d'anomalies musculaires parmi une pluralité prédéfinie de muscles de chaque sujet respectif de la pluralité de sujets, ladite génération du profil comprenant l'utilisation de fonctions non biaisées pour déterminer le profil, les fonctions non biaisées comprenant le regroupement hiérarchique de la pluralité de sujets, et ledit regroupement hiérarchique comprenant le regroupement hiérarchique multidimensionnel de la pluralité de sujets en fonction de la quantité ou du degré d'anomalie musculaire parmi une pluralité prédéfinie de muscles.

7. Système selon la revendication 6, ledit dispositif de traitement étant en outre configurer pour identifier, pour une anomalie musculaire, une quantité ou un degré de l'anomalie, ou ladite valeur moyenne étant une valeur normale moyenne correspondant à un muscle ou groupe de muscles normal respectif d'un ou plusieurs sujets de référence sans anomalie musculaire, ou ladite valeur moyenne correspondant à un muscle ou groupe de muscles d'un ou plusieurs sujets de référence, et au moins l'un desdits un ou plusieurs sujets présentant une quantité ou un degré d'anomalie musculaire différent d'au moins l'un desdits un ou plusieurs sujets de référence, ou ladite acquisition des données d'image comprenant l'acquisition de données d'imagerie par résonance magnétique (IRM) associées au muscle ou groupe de muscles sélectionné, ou ledit muscle ou groupe de muscles sélectionné étant sélectionné sur la base de la fonction correspondante avec laquelle le muscle ou groupe de muscles sélectionné opère pour lesdits un ou plusieurs sujets.

8. Système selon la revendication 6, ladite anomalie musculaire comprenant une hypertrophie ou une atrophie, et éventuellement :
ladite hypertrophie correspondant à un volume musculaire normalisé qui est supérieur à la valeur moyenne et ladite atrophie correspondant à un volume musculaire normalisé qui est inférieur à la valeur moyenne, ou ladite détermination de l'écart comprenant le calcul d'une quantité d'hypertrophie ou d'atrophie du muscle ou groupe de muscles sélectionné par rapport à la valeur moyenne.

9. Système selon la revendication 6, ledit profil est généré sur la base d'une quantité ou d'un degré d'anomalie musculaire correspondant au muscle ou groupe de muscles sélectionné de chacun de la pluralité de sujets, ou ladite génération du profil comprenant le regroupement de la pluralité de sujets en fonction de l'ampleur de l'anomalie musculaire respective.

10. Système selon la revendication 6, ladite fonction non biaisée comprenant en outre au moins l'une parmi : l'analyse des composants principaux ; et
la détermination du profil sur la base d'une relation avec des paramètres de performance ou de blessure associes à la pluralité de sujets.

11. Support non transitoire lisible par ordinateur stockant des instructions qui, lorsqu'elles sont exécutées par un ou plusieurs processeurs, amènent un dispositif informatique à exécuter un procédé qui comprend :

l'acquisition (302) de données d'image associées à un muscle ou groupe de muscles sélectionné d'un ou plusieurs sujets en recevant des données provenant d'un dispositif d'acquisition de données ou d'un dispositif de collecte, de stockage ou de traitement de données ;
la détermination (304), sur la base des données d'image, du volume musculaire du muscle ou groupe de muscles sélectionné ;
le calcul (306), sur la base du volume musculaire et de la taille et du poids desdits un ou plusieurs sujets, d'un volume musculaire normalisé en hauteur-poids pour le muscle ou groupe de muscles sélectionné ;
la détermination (308) d'un écart du volume musculaire normalisé en hauteur-poids du muscle ou groupe de muscles sélectionné à partir d'une valeur moyenne du volume musculaire associée à un muscle de référence ou groupe de muscles de référence correspondant ; et
l'identification (310), sur la base de l'écart, d'une anomalie musculaire ou de l'absence d'une anomalie musculaire dans le muscle ou groupe de muscles sélectionné, lesdits un ou plusieurs sujets comprenant une pluralité de sujets, chaque sujet possédant une anomalie musculaire dans le muscle ou groupe de muscles sélectionné respectif, et ledit procédé effectué par le dispositif informatique comprenant en outre la génération d'un profil indiquant un modèle d'anomalie musculaire parmi la pluralité de sujets, ladite génération du profil comprenant le regroupement de la pluralité de sujets sur la base de modèles particuliers d'anomalies musculaires parmi une pluralité prédéfinie de muscles de chaque sujet respectif de la pluralité de sujets, ladite génération du profil comprenant l'utilisation de fonctions non biaisées pour déterminer le profil, les fonctions non biaisées comprenant le regroupement hiérarchique de la pluralité de sujets, et ledit regroupement hiérarchique comprenant le regroupement hiérarchique multidimensionnel de la pluralité de sujets en fonction de la quantité ou du degré d'anomalie musculaire parmi une pluralité prédéfinie de muscles.

12. Support non transitoire lisible par ordinateur selon la revendication 11, ledit procédé effectué par le dispositif informatique comprenant en outre l'identification, pour une anomalie musculaire, d'une quantité ou d'un degré de l'anomalie, ou ladite valeur moyenne étant une valeur normale moyenne correspondant à un muscle ou groupe de muscles normal respectif d'un ou plusieurs sujets de référence sans anomalie musculaire, ou ladite valeur moyenne correspondant à un muscle ou groupe de muscles d'un ou plusieurs sujets de référence, et au moins l'un desdits un ou plusieurs sujets présentant une quantité ou un degré d'anomalie musculaire différent d'au moins l'un desdits un ou plusieurs sujets de référence, ou ladite acquisition des données d'image comprenant l'acquisition de données d'imagerie par résonance magnétique (IRM) associées au muscle ou groupe de muscles sélectionné, ou ledit muscle ou groupe de muscles sélectionné étant sélectionné sur la base de la fonction correspondante avec laquelle le muscle ou groupe de muscles sélectionné opère pour lesdits un ou plusieurs sujets.

13. Support non transitoire lisible par ordinateur selon la revendication 11, ladite anomalie musculaire comprenant une hypertrophie ou une atrophie, et éventuellement :
ladite hypertrophie correspondant à un volume musculaire normalisé qui est supérieur à la valeur moyenne et ladite atrophie correspondant à un volume musculaire normalisé qui est inférieur à la valeur moyenne, ou ladite détermination de l'écart comprenant le calcul d'une quantité d'hypertrophie ou d'atrophie du muscle ou groupe de muscles sélectionné par rapport à la valeur moyenne.

14. Support non transitoire lisible par ordinateur selon la revendication 11, ledit profil étant généré sur la base d'une quantité ou d'un degré d'anomalie musculaire correspondant au muscle ou groupe de muscles sélectionné de chacun de la pluralité de sujets, ou ladite génération du profil comprend le regroupement de la pluralité de sujets en fonction de l'ampleur de l'anomalie musculaire respective.

15. Support non transitoire lisible par ordinateur selon la revendication 11, lesdites fonctions non biaisées comprenant en outre au moins l'une parmi :

l'analyse des composants principaux ; et
la détermination du profil sur la base d'une relation avec des paramètres de performance ou de blessure associes à la pluralité de sujets.

**FIG. 1**

**FIG. 2**

## 300

ACQUIRE IMAGE DATA FOR SELECTED MUSCLE OR
GROUP OF MUSCLES
302

DETERMINE MUSCLE VOLUME BASED ON IMAGE DATA
304

CALCULATE HEIGHT-MASS NORMALIZED MUSCLE
VOLUME BASED ON DETERMINED MUSCLE VOLUME
AND SUBJECT HEIGHT AND MASS
306

DETERMINE DEVIATION OF HEIGHT-MASS NORMALIZED
MUSCLE VOLUME FROM MEAN VALUE
308

IDENTIFY PRESENCE OR ABSENCE OF MUSCLE
ABNORMALITY BASED ON DEVIATION
310

GENERATE PROFILE OF MUSCLE PATTERN
312

# FIG. 3

FIG. 4B

FIG. 4A

**FIG. 5**

3D Reconstruction of Legs

Standard Deviations away from Mean of Control Subjects

Zscore

Hypertrophy   Normal   Atrophy

FIG. 6

**A.)** Hypertrophy Z Scores

**B.)** muscle vectors in "athlete space"

**C.)** muscles clustered by euclidean distance

**D.)** Column Vectors

**E.)** athlete vectors in "muscle space"

**F.)** athletes clustered by euclidean distance

FIG. 7

EP 3 164 069 B1

FIG. 8

**FIG. 9**

FIG. 10

**FIG. 11**

FIG. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013023214 A1

### Non-patent literature cited in the description

- **MEYER, C. H. et al.** Simultaneous spatial and spectral selective excitation. *Magnetic Resonance in Medicine,* 1990, vol. 15, 287-304
- **HANDSFIELD, G. C. et al.** Relationships of 35 lower limb muscles to height and body mass quantified using MRI. *Journal of Biomechanics,* 2013
- **MEYER, C. H. et al.** Fast spiral coronary artery imaging. *Magnetic Resonance in Medicine,* 1992
- **CHEN, W. et al.** Fast conjugate phase image reconstruction based on a Chebyshev approximation to correct for B0 field inhomogeneity and concomitant gradients. *Magnetic Resonance in Medicine,* 2008